# EUROPEAN PATENT SPECIFICATION

(11) **EP 2 917 156 B1**
(45) Date of publication and mention of the grant of the patent: **25.11.2020**
(21) Application number: 13854016.6
(22) Date of filing: 02.10.2013
(51) Int. Cl.: C02F 11/04, C12M 1/107, C12P 5/02

(54) **METHOD OF INCREASING THE PRODUCTION OF BIOGAS FROM A WASTE ACTIVATED SLUDGE**
VERFAHREN ZUR VERMEHRUNG DER PRODUKTION VON BIOGAS AUS EINEM ABFALLAKTIVIERTEN SCHLAMM
PROCÉDÉ D'AUGMENTATION DE LA PRODUCTION DE BIOGAZ À PARTIR D'UNE BOUE DE DÉCHET ACTIVÉE

(30) Priority: 09.11.2012 SE 1251275
(43) Date of publication of application: 16.09.2015
(73) Proprietor: Sekab E-Technology AB, 891 26 Örnsköldsvik (SE)
(72) Inventor: LARSSON, Per, S375 32 Mörrum (SE); WINGREN, Anders, 414 75 Göteborg (SE)
(74) Representative: Kransell & Wennborg KB
(86) International application number: PCT/SE2013/051153
(87) International publication number: WO 2014/074048

(56) References cited:
- WO-A1-2011/006854
- WO-A2-2011/020000
- US-A- 4 017 421
- US-A1- 2008 193 994
- US-A1- 2008 193 994
- CONVERTI A ET AL: "CO-DIGESTION OF MUNICIPAL SEWAGE SLUDGES AND PRE-HYDROLYSED WOODY AGRICULTURAL WASTES", JOURNAL OF CHEMICAL TECHNOLOGY AND BIOTECHNOLOGY, WILEY, vol. 69, no. 2, 1 June 1997 (1997-06-01), pages 231-239, XP000696126, ISSN: 0268-2575
- ALDRICH ET AL.: 'Acidogenic Fermentation of Lignocellulosic Substrate with Activated Sludge.' CHEM. ENG. COMM. vol. 192, 2005, pages 1221 - 1242, XP055251980
- CONVERTI ET AL.: 'Co-digestion of Municipal Sewage Sludges and Pre-hydrolysed Woody Agricultural Wastes.' J. CHEM. TECH. BIOTECHNOL. vol. 69, 1997, pages 231 - 239, XP000696126
- TAHERZADEH ET AL.: 'Pretreatment of Lignocellulosic Wastes to Improve Ethanol and Biogas Production: A review.' INT. J. MOL. SCI. vol. 1008, no. 9, pages 1621 - 1651, XP002674403
- ESTEVEZ ET AL.: 'Effects of steam explosion and co-digestion in the methane production from Salix by mesophilic batch assays.' BIORESOURCE TECHNOLOGY vol. 104, 2012, pages 749 - 756, XP028351260
- DATABASE WPI Derwent Publications Ltd., London, GB; AN 2008-M35457, XP055260191 & CN 101 220 292 A (UNIV BEIJING CHEMICAL) 16 July 2008
- MARIA M ESTEVEZ ET AL: "Effects of steam explosion and co-digestion in the methane production fromby mesophilic batch assays", BIORESOURCE TECHNOLOGY, ELSEVIER, AMSTERDAM, NL, vol. 104, 5 November 2011 (2011-11-05), pages 749-756, XP028351260, ISSN: 0960-8524, DOI: 10.1016/J.BIORTECH.2011.11.017 [retrieved on 2011-11-15]
- MOHAMMAD J TAHERZADEH ET AL: "Pretreatment of lignocellulosic wastes to improve ethanol and biogas production: a review", INTERNATIONAL JOURNAL OF MOLECULAR SCIENCES, MOLECULAR DIVERSITY PRESERVATION INTERNATIONAL, BASEL, CH, vol. 9, no. 9, 1 September 2008 (2008-09-01), pages 1621-1651, XP002674403, ISSN: 1422-0067, DOI: 10.3390/IJMS9091621

## Description

### Technical field

The present invention relates to the field of biogas production. In particular it relates to a method of increasing the amount of biogas produced from a waste activated sludge (W.A.S.).

### Background

Waste water (e.g. sewage) treatment processes usually involves a preliminary treatment step wherein large and floating objects are removed from the waste water using some kind of screens. The waste water can thereafter be pumped to grit chambers where heavier inorganic material is allowed to settle out of the waste water to prevent damage to the pumps, and to prevent accumulation of such particles in the in sludge digesters. The preliminary treatment can be followed by a primary treatment including sedimentation of suspended solids and part of organic matter in a primary sedimentation tank (Jegatheesan et al.). The waste water from the primary treatment comprises suspended and solid colloidal organic matters. In conventional biological waste water treatment, these organic substances are taken up by microorganisms in a secondary treatment. The activated sludge process is one such type of secondary treatment. This process involves aeration of the primary treated sewage or wastewater in an aeration tank in the presence of microorganisms to develop a biological floc comprising several different species of saprotrophic bacteria, as well as different protozoan species, which metabolizes organic material and hence reduces the organic content of the waste water. When the wastewater has received sufficient treatment in the aeration tank, excess of mixed liquor (i.e. the mixture of waste water and microbial biomass) is transferred to a settling tank, which commonly is referred to as final clarifier or secondary settling tank, and the treated liquid fraction is run off to undergo further treatment before discharge. Part of the sludge settled in the final clarifier is returned to the aeration tank to re-seed the new wastewater entering the tank. This fraction of the activated sludge is known as return activated sludge (R.A.S.). Excess sludge is removed from the treatment process to keep the ratio of microorganisms to nutrients in the wastewater in balance. The excess sludge is commonly called waste activated sludge (W.A.S.) or surplus activated sludge (S.A.S.).

Converti et al. (JOURNAL OF CHEMICAL TECHNOLOGY AND BIOTECHNOLOGY, vol. 69, no. 2, p. 231-239), describes that material balance has been used to evaluate the COD behaviour and the time required for fed-batch digestion of mixtures of domestic sludges and pre-hydrolysed agricultural wastes. It is concluded that removal of lignin is necessary in order to carry out the continuous anaerobic digestion of pre hydrolysed agricultural wastes rich in woody materials.

Estevez et al. (BIORESOURCE TECHNOLOGY, vol. 104, p. 749-756), discloses that *Salix* that was steam exploded at different conditions of temperature and time was anaerobically digested in a series of batch tests. Steam explosion proved to be favorable to increase the methane yields up to 50%. Batch studies for mixtures of cow manure and steam exploded *Salix* were performed. A fraction up to 40% in volatile solids from pre-treated *Salix* provided good methane yields with a faster digestion process.

US 2008/0193994 discloses that solid organic waste is processed into a suitable feedstock that can be mixed with sewage for anaerobic digestion. The processing of the solid organic waste initially transforms the solid organic waste into a uniform biomass. The uniform biomass is then subjected to hydrolysis and volatile acid fermentation to dissolve the soluble compounds leaving a small residual solid component. The liquid in which the soluble compounds are dissolved is then mixed with sewage in an anaerobic digester to produce biogas including methane. The residual solid component can be composted.

Taherzadeh et al. (INTERNATIONAL JOURNAL OF MOLECULAR SCIENCES, vol. 9, no. 9, p. 1621-1651) is a review article that discloses that lignocelluloses are often a major or sometimes the sole components of different waste streams from various industries, forestry, agriculture and municipalities and that hydrolysis of these materials is the first step for either digestion to biogas or fermentation to ethanol. Methods that have been studied for pretreatment of lignocellulosic wastes for conversion to ethanol or biogas are reviewed.

WO 2011/020000 discloses an integrated anaerobic digester which converts a mixture of digestion effluent from a liquid anaerobic digester and a solid organic material (e.g., a lignocellulosic biomass, food waste, agricultural waste, etc.) to a biogas. The effluent of liquid anaerobic digester may be used as a nitrogen source and inoculum for solid state digestion.

US 4017421 discloses that aqueous solutions and suspensions of solid particles, even those containing less than one percent of organic materials, may be combusted with air, oxygen, or their mixtures in a process which provides for preheating in countercurrent batches of the raw original liquid by either open (direct contact) or closed (heat transfer surface) condensation of steam generated by multiple flash evaporations which cool earlier batches of hot liquid after the wet combustion. Waste liquids as sewage sludges and black liquors from wood pulping may have their organic constituents, as measured by BOD or COD, so reduced that the final, spent liquid may be discharged directly to a receiving body of water.

### Summary of the present disclosure

The treatment and disposal of W.A.S. represents a problem of growing importance of wastewater treatment plants all over the world and often account for a significant part of the plant's operating costs. To reduce the volume of the W.A.S. for disposal, anaerobic digesters can be used to convert W.A.S. to biogas. While anaerobic digesters are relatively effective at converting primary sludge to biogas, they are much less efficient at converting W.A.S. to biogas. The reason for this is that the cell membranes of the microbes in the W.A.S. are very resilient which limits the extent and rate of which the microbes are degraded in the anaerobic digesters. The present inventors have thus realized that there is a need to find improved methods for anaerobic degradation of W.A.S. to biogas. The present inventors have further realized that the biogas yield in a process of anaerobic degradation of W.A.S. can be increased if the load of biodegradable material is increased by co-digestion of a pre-treated lignocellulosic material together with the W.A.S.. This is highly beneficial since biogas is a valuable renewable energy resource.

The present inventors have surprisingly discovered that the amount of biogas produced from a co-digestion of a pretreated lignocellulosic material and W.A.S. can be increased if the lignocellulosic material is pretreated at high temperature (e.g. 120-250 °C) and at a low pH (e.g. 0.5 to 2.5) and if said pretreated lignocellulosic material is mixed with the W.A.S., and if the obtained mixture is incubated for a period of time prior to the anaerobic co-digestion. Therefore a first aspect of the present invention relates to a method for producing biogas comprising the following steps:
a) providing a lignocellulosic biomass
b) pretreating the lignocellulosic biomass at a temperature of 120-250 °C , preferably 140-230 °C, such as 170-220 °C and at a pH of 0.5 to 3.0, preferably at a pH of 1.0 to 2.5, such that a slurry of pretreated lignocellulosic biomass is obtained
c) providing a W.A.S. having a pH between 5.0 and 8.0, such as between 6.0 and 7.5
d) mixing the slurry of pretreated lignocellulosic biomass obtained in step b) with the W.A.S. provided in step c) such that a mixture comprising W.A.S. and pretreated lignocellulosic biomass is obtained
e) incubating the mixture obtained in step d) for at least 20 minutes such that a mixture of pretreated W.A.S. and pretreated lignocellulosic biomass is obtained
f) anaerobic digestion of the mixture of pretreated W.A.S. and pretreated lignocellulosic biomass obtained in e) by incubation of the mixture for a second incubation time at a temperature of 30-60 °C, preferably 35-55 °C preferably 35-40 °C in a digester comprising anaerobic microorganisms such that biogas and a digestate is produced,
   wherein the pH of the mixture obtained in step d) is 0.5-3.0 and wherein the temperature of the mixture obtained in step d) is 75-99 °C.

Further, the method comprises the steps of:
- adjusting the pH of the mixture of pretreated W.A.S. and pretreated lignocellulosic biomass obtained in e), prior to step f), such that the pH of the mixture of pretreated W.A.S. and pretreated lignocellulosic biomass is 6.0-8.0, preferably 6.5-7.5, such as 6.8-7.2; and
- adjusting the temperature of the mixture of pretreated W.A.S. and pretreated lignocellulosic biomass obtained in step e) to a temperature of 30-60 °C, preferably 35-55 °C preferably 35-40 °C, prior to step f).

A second, non-claimed, aspect of the invention relates to a system for production of biogas comprising the following:
a) unit for pretreatment of a lignocellulosic biomass having a biomass inlet and a pretreated biomass outlet
b) unit for sewage treatment having a sewage inlet and a W.A.S. outlet
c) mixing unit for mixing of pretreated lignocellulosic biomass with W.A.S. having an inlet for pretreated lignocellulosic biomass, an inlet for W.A.S. and a mixture outlet
d) unit for anaerobic digestion having a substrate inlet, a biogas outlet and a digestate outlet
   wherein:
   - the pretreated biomass outlet of the unit for pretreatment of a lignocellulosic biomass is connected to the inlet for pretreated lignocellulosic biomass of the mixing unit;
   - the W.A.S. outlet of the unit for sewage treatment is connected to the inlet for W.A.S. of the mixing unit; and
   - the mixture outlet of the mixing unit is connected to the substrate inlet of the unit for anaerobic digestion

### Brief description of the figures

Figure 1 shows two exemplary embodiments of the system for production of biogas, according to the present invention.
Figure 2 shows the results from an anaerobic digestion of a W.A.S. treated for 60 minutes at the following conditions prior to the anaerobic digestion:
   1) pH 6.25 at 20 °C
   2) pH 6.25 at 90 °C
   3) pH 2.0 at 20 °C
   4) pH 2.0 at 90 °C

The y-axis shows the amount of produced methane (Nm³ / tonᵥₛ) and the x-axis shows the retention time of the solids (days).

### Detailed description

The present inventors have surprisingly discovered that the amount of biogas produced from a co-digestion of a pretreated lignocellulosic material and W.A.S. can be increased if the lignocellulosic material is pretreated at high temperature (e.g. 120-250 °C) and at a low pH (e.g. 0.5 to 2.5) and if said pretreated lignocellulosic material is mixed with the W.A.S., and if the obtained mixture is incubated for a period of time prior to the anaerobic co-digestion. It appears that the pretreated lignocellulosic material has a "pretreating effect" on the W.A.S.. Without being bound by theory it is possible that chemical substances of the pretreated biomass contribute to the pretreating effect of the W.A.S. It is also possible that the temperature and pH of the pretreated ligocellulosic material at least partly is responsible for the observed effect. This is supported by the results presented in example 3 in the present application where a W.A.S. is treated at a temperature of 90 °C at a pH of 2.0. These conditions mimics the conditions in the mixture of W.A.S. and lignocellulosic biomass pretreated as described above. As shown in example 3, the treatment gave rise to an increased biogas production whereas treatment at 90 °C at pH 6.25 or at 20 °C at pH 2.0 did not have any positive effect on the biogas production. The combined low pH and high temperature of the pretreated lignocellulosic biomass thus seem to contribute to increasing the biogas yield from the W.A.S..

Thus a first aspect of the present invention relates to a method for producing biogas comprising the steps as claimed in appended claim 1.

In the present disclosure the mixture of W.A.S. and pretreated lignocellulosic biomass obtained after the incubation in step e) is referred to as "mixture of pretreated W.A.S. and pretreated lignocellulosic biomass". The rational behind this terminology is that the present inventors have discovered that the incubation of W.A.S. with a lignocellulosic biomass pretreated according to the present invention has a "pretreating effect" on the W.A.S., in the sense that more biogas can be generated from the W.A.S. in step f) compared to if the W.A.S. was introduced to the biogas digester without prior incubation with pretreated lignocellulosic biomass. Therefore the term "pretreated W.A.S." should not be interpreted as that the W.A.S. has been exposed to any other pretreatment besides the incubation with the pretreated lignocellulosic biomass in step e). However, the present invention does not exclude that the W.A..S. is exposed to other pretreatments as well.

Depending on how much pretreated W.A.S/pretreated lignocellulosic biomass, that are added to the digesters in step f), the pH of the mixture might needs to be adjusted to a pH suitable for anaerobic biogas production (i.e. 5.0-8.0) prior to step f). Therefore, the method further comprises a step of adjusting the pH of the mixture of pretreated W.A.S. and pretreated lignocellulosic biomass obtained in e), prior to step f), such that the pH of the mixture of pretreated W.A.S. and pretreated lignocellulosic biomass is 6.0-8.0, preferably 6.5-7.5, such as 6.8-7.2.

The optimal temperature in step f) depends on the anaerobic microorganisms present in the digester. If the microorganisms are thermophilic microorganisms the optimal temperature can for example be in the range 45-55 °C. In most cases the optimal temperature will however be about 37 °C , such as 35-40 °C. If the temperature of the mixture of pretreated W.A.S. and pretreated lignocellulosic biomass obtained in step e) is higher than the optimal temperature for step f) it can some times be necessary to adjust the temperature of the mixture of pretreated W.A.S. and pretreated lignocellulosic biomass obtained in step e). Thus the method further comprises a step of adjusting the temperature of the mixture of pretreated W.A.S. and pretreated lignocellulosic biomass obtained in step e) to a temperature of 30-60 °C, preferably 35-55 °C preferably 35-40 °C, prior to step f).

Generally, the buffering capacity of the W.A.S. is low and therefore the pH of the mixture comprising W.A.S. and pretreated lignocellulosic biomass obtained in step d) will be quite similar to the pH of the pretreated lignocellulosic biomass obtained in b). This is beneficial since a low pH, in combination with a high temperature contributes to the increased biogas yield from the W.A.S., see example 3. Therefore, the pH of the mixture obtained in step d) is 0.5-3.0, such as 0.5 -2.5, preferably 1.0-2.5, such as 1.0-2.0. In a most preferred embodiment the pH of the mixture obtained in step d) is 1.5-2.5. The temperature of the mixture obtained in step d) is 75-99 °C, such as 80-95 °C. One way of ensuring a relatively low pH and a relatively high temperature of the mixture obtained in d) is to prepare the mixture such that the mixture comprises a relatively high proportion of slurry of pretreated lignocellulosic biomass. Thus in one embodiment the mixture obtained in d) comprises at least 30 % (w/w) slurry of pretreated lignocellulosic biomass. In one embodiment the mixture obtained in d) comprises 30-90 % (w/w) slurry of pretreated lignocellulosic biomass and 10-70 % (w/w) W.A.S.. In one embodiment the mixture obtained in d) comprises 50-90 % (w/w) slurry of pretreated lignocellulosic biomass and 50-10 % (w/w) W.A.S.. In one embodiment the weight ratio of slurry of pretreated lignocellulosic biomass to W.A.S. in step d) is at least 3:2, preferably at least 2:1, preferably at least 3:1, such as at least 4:1.

The present inventors have observed that a relatively short incubation in step e) is sufficient for mediating an increased biogas yield from the W.A.S.. The first incubation time is at least 20 minutes such as at least 60 minutes. Generally the effect does not seem to increase after incubations over 300 minutes. Thus, in one embodiment the first incubation time is 20-300 minutes, such as 60-300 minutes. The second incubation time describes the average time the substrate (i.e. the mixture of pretreated W.A.S. and pretreated lignocellulosic biomass) remains in the digester. One measure of substrate residence time in the biogas digester is solids retention time which is the average time for the solids to remain in the biogas digester. In one embodiment the second incubation time is at least 24 h, such as at least 48 h, such as at least 120 h, such at least 240 h. In one embodiment the solids retention time of the biomass in the biogas digester in step f) is at least 24 h, such as at least 48 h, such as at least 120 h, such at least 240 h.

Biogas produced in anaerobic digester mainly consists of methane and carbon dioxide. A biogas comprising high amounts of methane is desirable due to the high energy value of methane. In advanced biogas digesters the proportion of methane in the gas can be 55-75% methane. Thus, in one embodiment the biogas produced in step f) comprises methane. In a preferred embodiment the biogas produced in step f) comprises at least 30 % (v/v) methane, such as at least 50 % (v/v).

Digestate produced in biogas digester can for example be used as soil conditioner or fertilizer. To reduce handling and transport costs, smell and the number of pathogens in the digestate it is desirable to dewater and dry the digestate. Therefore, in one embodiment the method is further comprising the step
g) dewatering the digestate to obtain a dewatered digestate;
   and in one embodiment the method comprises the step
h) drying the dewatered digestate obtained in g) such that a dried digestate is obtained. In one embodiment the digestate is dewatered to a TS of at least 10 %, such as to at least 15 % TS, such as to at least 20 % TS in step g) and in one embodiment the dewatered digestate is dried to a TS of at least 75 % such as to at least 85 % TS in step h).

Since the lignocellulosic biomass is converted to biogas in step f) it is desired that the pretreatment of the lignocellulosic biomass in step b) is as efficient as possible in terms of increasing the digestibility of the lignocellulosic biomass in the anaerobic digesters. The present inventors have realized that steam explosion using an acid catalyst is a particular suitable pretreatment method for increasing the anaerobic digestibility of lignocellulosic biomass in to biogas. Thus, in one preferred embodiment the method further comprises pretreating the lignocellulosic material under overpressure followed by releasing the pressure such that a slurry of pretreated lignocellulosic biomass and flash steam is obtained. Prior to the steam explosion the lignocellulosic biomass is preferably impregnated with an acidic impregnation chemical, followed by heating. The acidic impregnation chemical may be an acid solution, such as a mineral acid or an organic acid. The impregnation may also be performed with a gas, such as a sulfur dioxide gas or with the combination of a gas with a liquid to obtain e.g. sulfurous acid. Thus, in one embodiment step b) involves treatment of the lignocellulosic biomass with an acidic chemical. In one embodiment the acidic chemical is a mineral acid and/or an organic acid. In one embodiment the acidic chemical is selected from sulfur dioxide, sulfuric acid, sulfurous acid, acetic acid, and phosphoric acid. In a most preferred embodiment the acidic chemical is sulfuric acid. In a preferred embodiment the lingocellulosic biomass is impregnated with the acid chemical in a step prior to steam explosion of the lingocellulosic biomass.

Besides of the surprising increase in biogas yield achieved by incubating the W.A.S. with the pretreated lignocellulosic biomass slurry prior to anaerobic co-digestion, the present inventors have found other process solutions giving rise to synergistic effects by combining a steam explosion pretreatment facility with a biogas digester for digestion of W.A.S.. For example, the present inventors have realized that the heat energy released in the flash steam can be used to heat the W.A.S. which thus increases the biogas yield from the W.A.S.. This is particularly suitable if the mixture obtained in step d) has a temperature below 90 °C and even more suitable if the temperature of the mixture is below 75 °C. There are several different possibilities how to use the flash steam to heat the W.A.S.. For example the flash steam can be directed to a heat exchanger which indirectly heats the W.A.S. prior to mixing it with the pretreated biomass in step d). However it is usually more preferred to heat the mixture obtained in step d) and the heat exchanger can therefore be arranged to heat a vessel where the mixing in step d) takes place. It is however even more preferred that the flash steam, is used for direct heating of the mixture obtained in d), such that the flash steam in introduce in to the mixture. Thereby organic material released in the flash steam can be re-introduced to the biomass and thus converted to biogas in step f), which in turn increases the biogas yield from the process. Therefore, in one embodiment heat energy from the flash steam is used to heat the mixture obtained in step d). In one embodiment at least part of the obtained flash steam is introduced to the mixture comprising W.A.S. and pretreated lignocellulosic biomass obtained in step d), to the pretreated lignocellulosic biomass obtained in step b) and/or to the W.A.S. provided in c). In a preferred embodiment at least part of the obtained flash steam is introduced to the mixture comprising W.A.S. and pretreated lignocellulosic biomass obtained in step d).

As discussed above, the digestate can be used for example as fertilizer or soil conditioners but the feasibility and economic potential of this is dependent on efficient dewatering and drying of the digestate to reduce handling and transportation costs, as well as smell and pathogens number in the digestate. However, drying of a dewatered biomass is an expensive and energy consuming step and the present inventors have thus realized that there is a need for finding methods which decreases the drying costs. The inventors have further realized that the use of steam explosion as a pretreatment method for a lignocellulosic biomass, in connection with a biogas digestion plant, provides a possibility to reduce the costs of drying a digestate if the energy in the flash steam obtained in the pretreatment step is used in a drying step of a digestate. Therefore, in one preferred embodiment at least part of the obtained flash steam is used as a heating source in the drying of dewatered digestate in step h).

In the method according to the present invention any lignocellulosic biomass can be used as substrate. However lingocellulosic biomasses which need harsher pretreatment, such as for example wood materials are particularly suitable. Thus in one embodiment the lingocellulosic biomass provided in step a) is agricultural residues or wood material, such as wood chips. In a preferred embodiment the lignocellulosic biomass is hard wood and/or soft wood. In one embodiment the lignocellulosic biomass is spruce, pine, birch, oak, eucalyptus, switch grass, salix, banagrass, arundo, corn cobs, corn stover, oat hulls, sugar cane, bagasse, straw from barley, straw from wheat, straw from oat, straw from triticale and/or straw from rye.

W.A.S. is a sludge which to a large extent consists of microorganisms. A W.A.S. is retrieved from an activated sludge process. This process involves aeration of a sewage or wastewater in an aeration tank in the presence of microorganisms to develop a biological floc comprising several different species of saprotrophic bacteria. When the wastewater has received sufficient treatment in the aeration tank, excess of mixed liquor (i.e. the mixture of waste water and microbial biomass) is transferred to a settling tanks, which commonly is referred to as final clarifier or secondary settling tank, and the treated liquid fraction is run off to undergo further treatment before discharge. Part of sludge settled in the final clarifier is returned to the aeration tank to re-seed the new wastewater entering the tank. This fraction of the activated sludge is known as return activated sludge (R.A.S.). Excess sludge (W.A.S.) is removed from the treatment process to keep the ratio of microorganisms to nutrients in the wastewater in balance. Thus in one embodiment the W.A.S. has been prepared from an activated sludge process, wherein said process includes aeration of a sewage or wastewater in an aeration tank in the presence of microorganisms to develop a biological floc; transfer of an excess of mixed liquor to a settling tank and obtaining of the W.A.S. by removing of excess sludge from the settling tank. In one embodiment the method is further comprising a step
i) collecting biogas produced in step f) and optionally collecting a digestate produced in step f), g) and/or h).

A second, non-claimed, aspect of the invention relates to a system for production of biogas comprising the following:
a) unit for pretreatment of a lignocellulosic biomass having a biomass inlet and a pretreated biomass outlet
b) unit for sewage treatment having a sewage inlet and a W.A.S. outlet
c) mixing unit for mixing of pretreated lignocellulosic biomass with W.A.S. having an inlet for pretreated lignocellulosic biomass, an inlet for W.A.S. and a mixture outlet
d) unit for anaerobic digestion having a substrate inlet, a biogas outlet and a digestate outlet
   wherein:
   - the pretreated biomass outlet of the unit for pretreatment of a lignocellulosic biomass is connected to the inlet for pretreated lignocellulosic biomass of the mixing unit;
   - the W.A.S. outlet of the unit for sewage treatment is connected to the inlet for W.A.S. of the mixing unit; and
   - the mixture outlet of the mixing unit is connected to the substrate inlet of the unit for anaerobic digestion

When the W.A.S. has been mixed with pretreated lignocellulosic biomass slurry the pH of the mixture is typically below 3.0. If high amounts of the mixture are added to the unit for anaerobic digestion, without adjusting the pH to a pH more suitable for the microorganisms present in the unit for anaerobic digestion, there is a risk that the microorganisms are damaged and that the production of biogas will be decreased. That pH can preferably be adjusted in the mixing unit or in any other position in between the mixing unit and the unit for anaerobic digestion. Thus, the system is further comprising means for adjusting a pH of a mixture of pretreated lignocellulosic biomass and W.A.S. in the mixing unit and/or at a position downstream of the mixing unit but upstream of the unit for anaerobic digestion. The means for adjusting a pH of a mixture of pretreated lignocellulosic biomass and W.A.S. is arranged such that the pH in the mixing unit can be adjusted.

Since the pH in the mixture is acidic and the optimal pH in the unit for anaerobic digestion typically is about 7 the means for adjusting a pH needs to be capable of raising the pH of the mixture form an acidic pH to a neutral pH. Therefore, the means for adjusting a pH of a mixture of pretreated lignocellulosic biomass and W.A.S. is a vessel comprising an alkaline substance wherein said vessel has an outlet connected to an inlet for alkaline substance of the mixing unit. The pH can also be adjusted at any stage after the mixing unit but prior to addition of the mixture to the unit for anaerobic digestion. Thus, the vessel comprising an alkaline substance has an outlet connected to a position of the system downstream of the mixing unit but upstream of the unit for anaerobic digestion. In this respect the term downstream and upstream is in relation to the biomass flow in the system.

The alkaline substance can be any alkaline substance capable of raising the pH of the mixture of pretreated lignocellulosic biomass and W.A.S to a neutral pH. Preferably the alkaline substance is an alkaline solution such as sodium hydroxide or lime. The alkaline substance may be calcium carbonate, calcium hydroxide or calcium oxide.

The unit for pretreatment of a lignocellulosic biomass comprises a pressure reactor comprising the biomass inlet and a biomass outlet and wherein the unit for pretreatment further comprises a flashing chamber for recovery of flash steam from the lignocellolosic biomass from the pressure reactor, and wherein said flashing chamber is comprising an inlet being connected to the outlet of the pressure reactor, a flash steam outlet and the pretreated biomass outlet. The present inventors have realized that the generated flash steam can be used to heat the W.A.S, and/or the mixture of W.A.S and pretreated lingocellulosic biomass obtained in step d) of the method according to the present invention. This is beneficial since the high temperature, in combination with a low pH, seems to contribute to increasing the biogas production from the mixture obtained in step d). The inventors have further realized that the flash steam comprises organic substances that can be re-introduced in step d) such that said organic compounds can be converted into biogas in step f). Thereby, two problems can be solved by introducing flash steam into the mixing unit:
1) the temperature is increased which leads to a more efficient pretreatment of the W.A.S.
2) The amount of organic compounds in step f) is increased.
   Both of theses feature increases the biogas yield from the process.

Therefore, the flash steam outlet is connected to a flash steam inlet of the mixing unit. Alternatively, the flash steam outlet is connected to a heat exchanger and said heat exchanger is arranged to transfer heat from the flash steam to the mixing unit. This is however less preferred since the organic compounds in the flash steam is not utilized for production of biogas.

The system further comprises a digestate dewatering unit having
- a digestate inlet connected to the digestate outlet of the unit for anaerobic digestion,
- a liquid fraction outlet and
- an outlet for dewatered digestate

The dewatering unit comprises a filter or a centrifuge. The dewatering unit comprises a filter press or a decanter centrifuge. The disclosed system may further comprise a digestate drying unit having
- an inlet for dewatered digestate connected to the outlet for dewatered digestate of the digestate dewatering unit;
- an outlet for dried digestate;

As discussed above the energy from the flash steam can also be used for drying of a dewatered digestate. The digestate drying unit therefore has a flash steam inlet connected to the flash steam outlet of the flashing chamber. The digestate can also be dried by indirect means using a heat exchanger. Therefore, the flash steam outlet of the flashing chamber is connected to a heat exchanger and said heat exchanger is arranged to transfer heat from the flash steam to the digestate drying unit.

In the present disclosure, the term "primary sludge inlet" can be used interchangeable with the term "sewage inlet". Then term should nevertheless be interpreted as an inlet for sewage.

The optimal temperature in the unit for anaerobic digestion depends on the anaerobic microorganisms present in the unit for anaerobic digestion. If the temperature of the mixture of pretreated W.A.S. and pretreated lignocellulosic biomass in the mixing unit is higher than the optimal temperature in the unit for anaerobic digestion it can some times be necessary to adjust the temperature of the mixture of pretreated W.A.S. and pretreated lignocellulosic biomass prior to introduction of the mixture in the unit for anaerobic digestion. This is particularly beneficial when large amounts of the mixture of pretreated W.A.S. and pretreated lignocellulosic is added to the unit for anaerobic digestion. Therefore the system preferably comprises a unit for adjusting a temperature of a mixture of pretreated lignocellulosic biomass and W.A.S. The said unit for adjusting a temperature is arranged at a position such that the temperature of the mixture can be adjusted in the mixing unit and/or at a position downstream of the mixing unit but upstream of the unit for anaerobic digestion. In a preferred embodiment the unit for adjusting a temperature of a mixture of pretreated lignocellulosic biomass and W.A.S is a heat exchanger. The heat exchanger is arranged to adjust a temperature of a mixture in the mixing unit. The heat exchanger is arranged to adjust the temperature of the mixture at a position downstream of the mixing unit but upstream of the unit for anaerobic digestion

The unit for sewage treatment is a sewage treatment plant or a waste water treatment plant. In one embodiment the sewage treatment plant is an activated sludge plant.

### Detailed description of exemplary embodiments

### Example 1

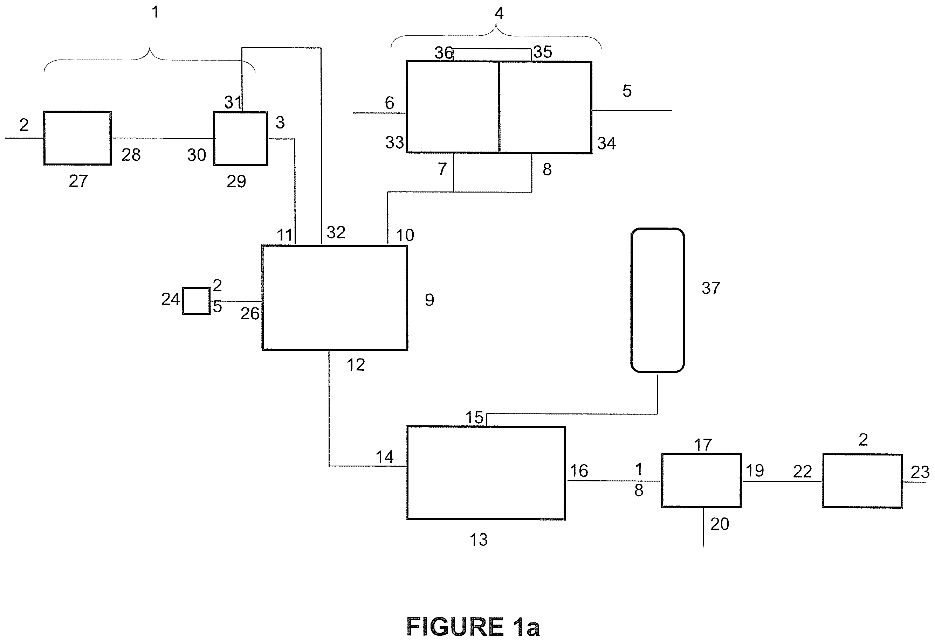

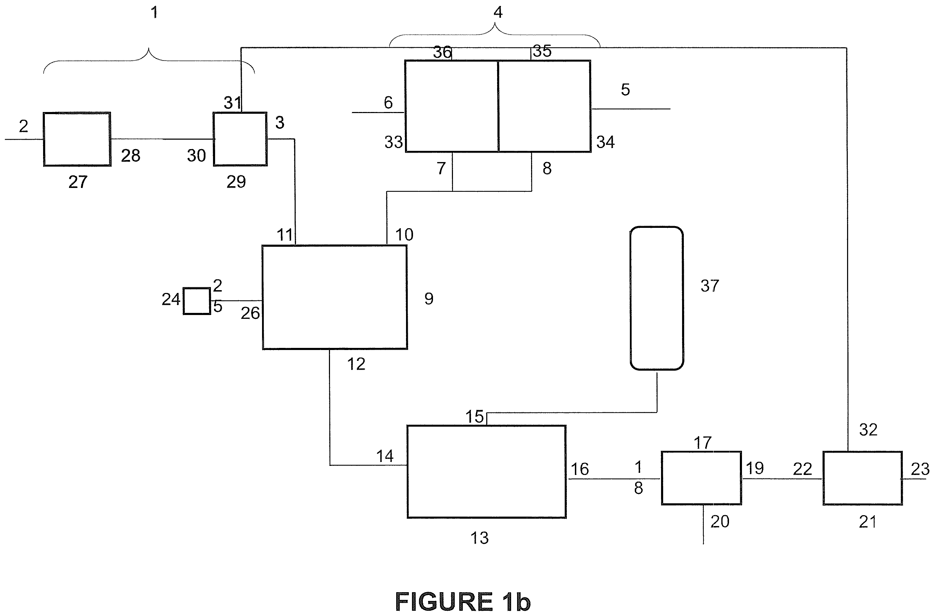

Figure 1 a shows an exemplary embodiment of the System for production of biogas according to the present disclosure. The system comprises a unit for pretreatment of a lignocellulosic biomass (1) having a biomass inlet (2) and a pretreated biomass outlet (3). The system further comprises a unit for sewage treatment (4) having a sewage inlet (5), an outlet for purified waste water (6) and a W.A.S. outlet (7). Furthermore, the system comprises a mixing unit (9) for mixing of pretreated lignocellulosic biomass with W.A.S. and a unit for anaerobic digestion (13). The mixing unit (9) has an inlet for pretreated lignocellulosic biomass (11) an inlet for W.A.S (10) and a mixture outlet (12). The unit for anaerobic digestion (13) comprises a substrate inlet (14), a biogas outlet (15) and a digestate outlet (16).The pretreated biomass outlet (3) of the unit for pretreatment of a lignocellulosic biomass (1) is connected to the inlet for pretreated lignocellulosic biomass (11) of the mixing unit (9). The W.A.S. outlet (7) of the unit for sewage treatment (4) is connected to the inlet for W.A.S. (10) of the mixing unit (9) and the mixture outlet (12) of the mixing unit (9) is connected to the substrate inlet (14) of the unit for anaerobic digestion (13). The unit for sewage treatment (4) can be, or constitute a part of an activated sludge plant comprising an aeration tank (34) and a final clarifier tank (33). Primary treated waste water is aerated in the aeration tank (34) in the presence of microorganisms to develop a biological floc comprising several different species of saprotrophic bacteria. When the wastewater has received sufficient treatment in the aeration tank (34), excess of mixed liquor (i.e. the mixture of waste water and microbial biomass) is transferred through a mixed liquor outlet (35) of the aeration tank (34) and introduced in to the final clarifier tank (33) through a mixed liquor inlet (36) of the final clarifier tank (36). The treated liquid fraction is run off through the outlet for purified waste water (6) to undergo further treatment before discharge. Activated sludge settled in the final clarifier tank (33) exits the final clarifier tank (33) through the W.A.S. outlet (7). A part of this sludge is returned to the aeration tank (34) via an R.A.S. inlet (8) of the aeration tank (34) to re-seed the new wastewater entering the aeration tank (34). Excess sludge (W.A.S.) is removed from the treatment process by introduction in to the mixing unit (9) via the inlet for W.A.S (10). In the mixing unit (10) the W.A.S. is mixed with pretreated lignocellulosic biomass entering the mixing unit through the inlet for pretreated lignocellulosic biomass (11) of the mixing unit (9). The mixing of the two different substrates within the mixing unit (9) converts the W.A.S to a better substrate for anaerobic digestion. Prior to introduction of the mixture of W.A.S and pretreated lignocellulosic biomass in to the unit for anaerobic digestion (13) the pH of the mixture is increased from about 2.0 to about 7.0 such that the pH in the unit for anaerobic digestion (13) will remain about 7 after addition of the mixture. Therefore the system further comprises means for adjusting a pH (24) having an outlet for alkaline substance (25) connected to an inlet for alkaline substance (26) of the mixing unit (9). Biogas produced in the unit for anaerobic digestion (13) exits the unit for anaerobic digestion (13) through the outlet for biogas (15) and is directed to a biogas storage tank (37). The digestate exits the unit for anaerobic digestion (13) through the digestate outlet (16) and enters a digestate dewatering unit (17) through a digestate inlet. In the digestate dewatering unit a liquid fraction is separated from the digestate using a filter press and the liquid fraction is run off through a liquid fraction outlet (20). This results in a dewatering of the digestate to a TS of 25 %. The dewatered digestate thereafter exits the dewatering unit through an outlet for dewatered digestate (19) whereupon it enters a digestated drying unit (21) through an inlet for dewatered digestate (22). The dewatered digestate is dried to a 95 % TS in the digestated drying unit (21) and thereafter exits the drying unit through an outlet for dried digestate (23). The unit for pretreatment of a lignocellulosic biomass (1) comprises a pressure reactor (27), comprising the biomass inlet (2) and a biomass outlet (28), and a flashing chamber (29) for recovery of flash steam from the lignocellolosic biomass from the pressure reactor (27). The flashing chamber (29) is comprising an inlet (30) being connected to the outlet of the pressure reactor (28), a flash steam outlet (31) and the pretreated biomass outlet (3). The flash steam outlet (31) is connected to a flash steam inlet (32) of the mixing unit (9). Thereby the heat energy in the flash steam can be used to heat the mixture of W.A.S. and pretreated lignocellulosic biomass in the mixing unit (9). Furthermore, organic compounds in the flash steam also mixes with the mixture of W.A.S and pretreated lignocellulosic biomass in the mixing unit (9) and is thus thereafter introduced into the unit for anaerobic digestion (13) and thus the organic compounds are used as a substrate in the anaerobic digestion which in turn increases the biogas yield.

### Example 2

Figure 1 b shows another example of the system for production of biogas according to the present disclosure which is similar to the embodiment described in example 1. The flash steam outlet (31) of the flashing chamber (29) is connected to a flash steam inlet (32) of the digestated drying unit (21). Thereby the energy in the flash steam can be utilized in the drying of digestate in the digestated drying unit (21).

### Example 3

300 ml of WAS was acidified to pH 2.0 using 2M HCI solution. A total of 29 ml of HCI solution was added to the sludge. As control, 300 ml of sludge was not acidified but kept at original pH (6.25).

Half of both acidified and control sludge was transferred to plastic bottles with a non gas tight lid and stored at room temperature for ca 6 hours.

Half of both acidified and control sludge was transferred to plastic bottles with a non gas tight lid and heated for 120 min in an oven at 105°C with intermittent manual stirring. When the temperature reached 90°C the bottles were transferred to a 90°C oven and stored therein for 60 minutes, with manual stirring every 15 minutes. Thereafter the bottles were allowed to cool to room temperature and the pH was adjusted to 7.0 by addition of sodium hydroxide. This was followed by anaerobic digestion for 62 days at 37°C in 300 ml gas-tight serum bottles containing 100 ml liquid volume (inoculum, substrate, salts and water). The gas-phase was N2:CO2 (80:20). Total gas-and methane production was measured at eight occasions over the incubation period.

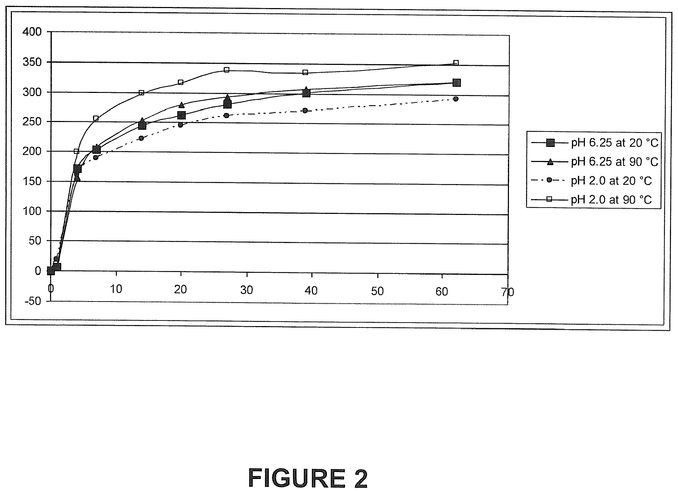

As shown in figure 2, treatment of W.A.S. at pH 2.0 at 90 °C gives rise to an increased biogas production whereas treatment at 90 °C at pH 6.25; or at 20 °C at pH 2.0 does not have any positive effect on the biogas production.

### REFERENCES

Jegatheesan V. et al. 2005, "Advances in Biological wastewater treatment" Biology, 21: 375-382

## Claims

1. Method for producing biogas comprising the following steps:
a) providing a lignocellulosic biomass
b) pretreating the lignocellulosic biomass at a temperature of 120-250 °C , preferably 140-230 °C, such as 170-220 °C and at a pH of 0.5 to 3.0, preferably at a pH of 1.0 to 2.5, such that a slurry of pretreated lignocellulosic biomass is obtained
c) providing a waste activated sludge (W.A.S.) having a pH between 5.0 and 8.0, such as between 6.0 and 7.5
d) mixing the slurry of pretreated lignocellulosic biomass obtained in step b) with the W.A.S. provided in step c) such that a mixture comprising W.A.S. and pretreated lignocellulosic biomass is obtained
e) incubating the mixture obtained in step d) for at least 20 minutes such that a mixture of pretreated W.A.S. and pretreated lignocellulosic biomass is obtained
f) anaerobic digestion of the mixture of pretreated W.A.S. and pretreated lignocellulosic biomass obtained in e) by incubation of the mixture for a second incubation time at a temperature of 30-60 °C, preferably 35-55 °C preferably 35-40 °C in a digester comprising anaerobic microorganisms such that biogas and a digestate is produced,
wherein the pH of the mixture obtained in step d) is 0.5-3.0 and wherein the temperature of the mixture obtained in step d) is 75-99 °C and
wherein the method further comprises the steps of
- adjusting the pH of the mixture of pretreated W.A.S. and pretreated lignocellulosic biomass obtained in e), prior to step f), such that the pH of the mixture of pretreated W.A.S. and pretreated lignocellulosic biomass is 6.0-8.0, preferably 6.5-7.5, such as 6.8-7.2 and
- adjusting the temperature of the mixture of pretreated W.A.S. and pretreated lignocellulosic biomass obtained in step e) to a temperature of 30-60 °C, preferably 35-55 °C preferably 35-40 °C, prior to step f).

2. Method according to claim 1 wherein the mixture obtained in d) comprises 30-90 % (w/w) slurry of pretreated lignocellulosic biomass and 10-70 % (w/w) W.A.S.

3. Method according to claim 1 or 2 wherein step b) comprises pretreating the lignocellulosic material under overpressure followed by releasing the pressure such that a slurry of pretreated lignocellulosic biomass and flash steam is obtained

4. Method according to claim 3 wherein heat energy from the flash steam is used to heat the mixture obtained in step d)

5. Method according to any of the claims 3-4 wherein at least part of the obtained flash steam is introduced to the mixture comprising W.A.S. and pretreated lignocellulosic biomass obtained in step d) or to the W.A.S. provided in step c)

## Patentansprüche

1. Verfahren zum Erzeugen von Biogas, umfassend die folgenden Schritte:
a) Bereitstellen einer Lignocellulose-Biomasse,
b) Vorbehandeln der Lignocellulose-Biomasse bei einer Temperatur von 120 bis 250 °C, vorzugsweise bei 140 bis 230 °C, zum Beispiel bei 170 bis 220 °C, und bei einen pH-Wert von 0,5 bis 3,0, vorzugsweise bei einem pH-Wert von 1,0 bis 2,5, derart, dass eine Aufschlämmung aus vorbehandelter Lignocellulose-Biomasse erhalten wird,
c) Bereitstellen eines Überschussschlammes (Waste Activated Sludge, W.A.S.) mit einem pH-Wert zwischen 5,0 und 8,0, zum Beispiel zwischen 6,0 und 7,5,
d) Mischen der in Schritt b) erhaltenen Aufschlämmung aus vorbehandelter Lignocellulose-Biomasse mit dem in Schritt c) bereitgestellten W.A.S., derart, dass eine Mischung umfassend W.A.S. und vorbehandelte Lignocellulose-Biomasse erhalten wird,
e) Inkubieren der in Schritt d) erhaltenen Mischung für mindestens 20 Minuten, derart, dass eine Mischung aus vorbehandeltem W.A.S. und vorbehandelter Lignocellulose-Biomasse erhalten wird,
f) anaerober Aufschluss der in e) erhaltenen Mischung aus vorbehandeltem W.A.S. und vorbehandelter Lignocellulose-Biomasse durch Inkubation der Mischung für eine zweite Inkubationszeit bei einer Temperatur von 30 bis 60 °C, vorzugsweise bei 35 bis 55 °C, vorzugsweise bei 35 bis 40 °C, in einer Vergärungsanlage, die anaerobe Mikroorganismen umfasst, derart, dass Biogas und ein Gärrest hergestellt werden,
wobei der pH-Wert der in Schritt d) erhaltenen Mischung 0,5 bis 3,0 beträgt und wobei die Temperatur der in Schritt d) erhaltenen Mischung 75 bis 99 °C beträgt,
und
wobei das Verfahren ferner die folgenden Schritte umfasst:
- Einstellen des pH-Werts der in e) erhaltenen Mischung aus vorbehandeltem W.A.S. und vorbehandelter Lignocellulose-Biomasse vor Schritt f), derart, dass der pH-Wert der Mischung aus vorbehandeltem W.A.S. und vorbehandelter Lignocellulose-Biomasse 6,0 bis 8,0, vorzugsweise 6,5 bis 7,5, zum Beispiel 6,8 bis 7,2, beträgt, und
- Einstellen der Temperatur der in Schritt e) erhaltenen Mischung aus vorbehandeltem W.A.S. und vorbehandelter Lignocellulose-Biomasse auf eine Temperatur von 30 bis 60 °C, vorzugsweise von 35 bis 55 °C, vorzugsweise von 35 bis 40 °C, vor Schritt f).

2. Verfahren nach Anspruch 1, wobei die in d) erhaltene Mischung 30 bis 90 % (Gew./Gew.) Aufschlämmung aus vorbehandelter Lignocellulose-Biomasse und 10 bis 70 % (Gew./Gew.) W.A.S. umfasst.

3. Verfahren nach Anspruch 1 oder 2, wobei Schritt b) das Vorbehandeln des Lignocellulose-Materials unter Überdruck umfasst, gefolgt vom Ablassen des Drucks, derart, dass eine Aufschlämmung aus vorbehandelter Lignocellulose-Biomasse und Entspannungsdampf erhalten wird.

4. Verfahren nach Anspruch 3, wobei Wärmeenergie aus dem Entspannungsdampf verwendet wird, um die in Schritt d) erhaltene Mischung zu erwärmen.

5. Verfahren nach einem der Ansprüche 3 bis 4, wobei mindestens ein Teil des erhaltenen Entspannungsdampfes in die in Schritt d) erhaltene Mischung umfassend W.A.S. und vorbehandelte Lignocellulose-Biomasse oder in den in Schritt c) bereitgestellten W.A.S. eingeführt wird.

## Revendications

1. Procédé de production de biogaz comprenant les étapes suivantes consistant à :
a) fournir une biomasse lignocellulosique,
b) prétraiter la biomasse lignocellulosique à une température de 120 à 250 °C, de préférence 140 à 230 °C, tel que 170 à 220 °C et à un pH de 0,5 à 3,0, de préférence à un pH de 1,0 à 2,5, de telle manière qu'une solution épaisse de biomasse lignocellulosique prétraitée est obtenue,
c) fournir une boue activée de déchets (W.A.S.) ayant un pH compris entre 5,0 et 8,0, tel qu'entre 6,0 et 7,5,
d) mélanger la solution épaisse de biomasse lignocellulosique prétraitée obtenue dans l'étape b) avec la W.A.S. fournie dans l'étape c) de telle manière qu'un mélange comprenant la W.A.S. et la biomasse lignocellulosique est obtenu,
e) incuber le mélange obtenu dans l'étape d) durant au moins 20 minutes de telle manière qu'un mélange de W.A.S. prétraitée et de biomasse lignocellulosique prétraitée est obtenu,
f) digérer de manière anaérobie le mélange de W.A.S. prétraitée et de biomasse lignocellulosique prétraitée obtenu dans e) pour un second temps d'incubation à une température de 30 à 60 °C, de préférence 35 à 55 °C de préférence 35 à 40 °C dans un digesteur comprenant des microorganismes anaérobies tels qu'un biogaz et un digestat est produit,
le pH du mélange obtenu dans l'étape d) étant de 0,5 à 3,0 et la température du mélange obtenu dans l'étape d) étant de 75 à 99 °C et
le procédé comprenant en outre les étapes de
- ajuster le pH du mélange de la W.A.S prétraitée et de la biomasse lignocellulosique prétraitée obtenu dans e), avant l'étape f), de telle manière que le pH de la W.A.S. prétraitée et de la biomasse lignocellulosique prétraitée est de 6,0 à 8,0, de préférence de 6,5 à 7,5 tel que de 6,8 à 7,2 et
- ajuster la température du mélange de W.A.S. prétraitée et de biomasse lignocellulosique prétraitée obtenu dans e) à une température de 30 à 60 °C, de préférence de 35 à 55 °C de préférence de 35 à 40 °C, avant l'étape f).

2. Procédé selon la revendication 1, dans lequel le mélange obtenu dans d) comprend 30 à 90 % (p/p) de solution épaisse de biomasse lignocellulosique prétraitée et 10 à 70 % (p/p) de W.A.S.

3. Procédé selon la revendication 1 ou 2, dans lequel l'étape b) comprend le prétraitement du matériau lignocellulosique sous surpression suivi par la libération de la pression de telle manière qu'une solution épaisse de biomasse lignocellulosique prétraitée et de vapeur instantanée est obtenue.

4. Procédé selon la revendication 3, dans lequel l'énergie de chaleur de la vapeur instantanée est utilisée pour chauffer le mélange obtenu dans l'étape d) .

5. Procédé selon l'une quelconque des revendications 3 à 4, dans lequel au moins une partie de la vapeur instantanée obtenue est introduite dans le mélange comprenant la W.A.S. et la biomasse lignocellulosique prétraitée obtenue dans l'étape d) ou dans la W.A.S. fournie dans l'étape c).
